# FASCICULE DE BREVET EUROPEEN

(11) **EP 2 237 730 B1**
(45) Date de publication et mention de la délivrance du brevet: **12.08.2015**
(21) Numéro de dépôt: 09720279.0
(22) Date de dépôt: 02.02.2009
(51) Int. Cl.: A61B 17/60, A61B 17/80

(54) **DISPOSITIF D'OSTEOSYNTHESE A MOYENS DE FIXATION RAPIDES**
OSTEOSYNTHESEVORRICHTUNG MIT SCHNELLBEFESTIGUNGSMITTELN
OSTEOSYNTHESIS DEVICE WITH RAPID FIXING MEANS

(30) Priorité: 01.02.2008 FR 0800556
(43) Date de publication de la demande: 13.10.2010
(73) Titulaire: Worcel, Alexandre, Parc de Mongarny 95680 Montlignon (FR); Worcel, Manuel, 75012 Paris (FR); Worcel, Julia, 95680 Montlignon (FR); Worcel, Marie, 95680 Montlignon (FR); Paoli, Albert, 95300 Pontoise (FR); Paoli, Myriam, 95300 Pontoise (FR); Paoli, Edouard, 95300 Pontoise (FR); Paoli, Jean, 95300 Pontoise (FR); Lombardo-Fiault, Bernard, 75011 Paris (FR); Laumonier, Alain, 95690 Nesles La Vallée (FR); Laumonier, Rémi, 95300 Pontoise (FR); Laumonier, Bruno, 95690 Nesles La Vallée (FR); Laumonier, Yves, 95690 Nesles La Vallée (FR); Laumonier, Nicolas, 95300 Pontoise (FR); Ateliers Laumonier, SAS, 95690 Nesles La Vallée (FR)
(72) Inventeur: Worcel Alexander, 95680 Montlignon (FR)
(74) Mandataire: Gendron, Vincent Christian
(86) Numéro de dépôt international: PCT/FR2009/000116
(87) Numéro de publication internationale: WO 2009/112705

(56) Documents cités:
- EP-A- 0 517 939
- WO-A-2007/138062
- US-A1- 2005 187 551
- US-A1- 2005 187 552

## Description

La présente invention se rapporte à un dispositif d'ostéosynthèse comprenant une plaque et des moyens de fixation permettant de maintenir une broche en travers de ladite plaque.

Un domaine d'application envisagé est notamment celui de la réduction des fractures pour lequel la plaque est étendue le long de deux parties osseuses délimitées par la fracture et au moins deux broches sont installées à travers la plaque et respectivement vissée dans lesdites deux parties osseuses. De la sorte, les deux broches étant maintenues en position fixe l'une par rapport à l'autre par l'intermédiaire de la plaque, les deux parties osseuses sont corrélativement maintenues ensemble l'une contre l'autre. Un tel dispositif est généralement installé durant une période définie, de l'ordre du mois, pendant laquelle les deux parties osseuses se ressoudent ensemble.

De nombreux dispositifs de ce type sont déjà connus, la plaque présente alors au moins deux évidements pratiqués dans son épaisseur et ils la traversent de part en part. Ils sont espacés l'un de l'autre de manière à pouvoir être respectivement ajustés en regard des deux parties osseuses. Les évidements sont adaptés à recevoir les moyens de fixation du type précité. Ils comprennent généralement une couronne ou une bague, montée vissée dans l'évidement. Et la broche est montée à travers cette bague qui la guide selon une direction déterminée par rapport à la plaque, par exemple sensiblement inclinée par rapport à une perpendiculaire à la plaque. Le document EP 1 583 478 par exemple, divulgue un tel dispositif, et la broche est maintenue en position fixe par rapport à la plaque, par l'intermédiaire d'une douille de blocage à l'intérieur de laquelle la broche est engagée, et qui vient se visser à l'intérieur de la bague, autour de la broche, selon un axe décalé angulairement par rapport à l'axe de la broche. Ainsi, compte tenu de ce décalage angulaire, le visage de la douille de blocage provoque progressivement le blocage irréversible de la broche par rapport à la plaque.

Ce dispositif est performant, lorsque la broche s'étend dans une direction sensiblement perpendiculaire à la plaque ou avec un décalage angulaire relativement faible par rapport à ladite direction perpendiculaire. En revanche, le maintien en position fixe d'une broche inclinée en travers d'une plaque est relativement malaisé à réaliser. En outre, la broche doit être ajustée précisément dans la douille de blocage et elle-même doit être ajustée dans la bague avec des tolérances extrêmement faibles. Aussi, l'usinage de telles pièces requiert des machines-outils spéciales et surtout, des temps de réalisation relativement longs par rapport aux autres pièces mécaniques du même type. Par conséquent, leur coût en est parallèlement augmenté.

EP 0 517 939 divulgue également un dispositif comportant les caractéristiques techniques du préambule de la revendication 1.

Aussi, un problème qui se pose et que vise à résoudre la présente invention, est de proposer un dispositif d'ostéosynthèse qui permette non seulement de maintenir une broche en position fixe en travers d'une plaque selon des directions inclinées par rapport à la plaque, bien évidemment avec une grande rigidité, mais aussi qui soit relativement bon marché à réaliser.

Dans le but de résoudre ce problème, la présente invention propose un dispositif d'ostéosynthèse comprenant une plaque apte à être étendue le long d'une pièce osseuse et des moyens de fixation pour maintenir une broche en travers de ladite plaque, ladite broche étant vissable dans ladite pièce osseuse, ladite plaque présentant un évidement pratiqué dans son épaisseur pour recevoir lesdits moyens de fixation, ledit évidement définissant un bord interne fermé, lesdits moyens de fixation comprenant une couronne destinée à recevoir ladite broche, ladite couronne étant adaptée à venir en prise avec ledit bord interne, et lesdits moyens de fixation comprenant en outre, des moyens de blocage vissables pour bloquer ladite broche en position fixe par rapport à ladite plaque ; selon l'invention, ladite plaque présente au moins un orifice taraudé pratiqué en bordure dudit évidement de manière à présenter une ouverture axiale débouchant dans ledit bord interne, tandis que ladite couronne est fendue radialement de manière à être diamétralement compressible;et lesdits moyens de blocage vissables comprennent une vis destinée à être vissée à travers ledit orifice taraudée pour venir prendre appui contre ladite couronne à travers ladite ouverture axiale et provoquer à la fois, l'appui à force de ladite couronne de guidage contre une portion dudit bord interne diamétralement opposée audit orifice taraudé, et le resserrement de ladite couronne sur ladite broche, par quoi ladite broche est maintenue en position fixe par rapport à ladite plaque.

Ainsi, une caractéristique de l'invention réside dans le mode de coopération de la couronne, apte à être logée dans l'évidement, et de la broche qui est alors engagée en travers de la couronne, et grâce au concours des moyens de blocage vissables, lesquels sous forme de vis, sont vissées dans un orifice taraudé qui débouche partiellement dans l'évidement, et viennent prendre appui contre la couronne au fur et à mesure du vissage. De la sorte, non seulement la couronne est entraînée contre le bord interne de l'évidement, selon une direction sensiblement parallèle à la plaque et dans un sens diamétralement opposé à la vis, mais au surplus, grâce à sa fente radiale, la couronne peut se resserrer autour de la broche en la coinçant. Par conséquent, la couronne est en prise entre la vis qui pénètre dans l'évidement, et le bord interne opposé de ce dernier, tandis que la broche est, elle, en prise dans la couronne. La broche est donc maintenue en position fixe par rapport à ladite plaque. Ainsi qu'on l'expliquera de manière plus détaillée dans la suite de la description, on s'affranchit ici du lien vis/écrou entre la couronne et la plaque. En effet, la couronne peut alors être maintenue dans une position telle, que le plan moyen qu'elle définit soit incliné par rapport à la plaque. Et partant, la broche pourra être maintenue en position fixe en travers de la plaque selon des inclinaisons relativement importantes.

Avantageusement, les moyens de blocage vissables sont constitués d'une vis tronconique évasée de la pointe vers la tête. De la sorte, au fur et à mesure de son vissage dans l'orifice taraudé, la vis s'enfonce latéralement par rapport à son axe de vissage, à travers l'ouverture axiale vers l'intérieur de l'évidement pratiqué dans la plaque, et vient prendre appui contre la couronne en y exerçant un effort qui s'accroît. Au surplus, l'orifice taraudé étant de symétrie sensiblement cylindrique, la vis tronconique exerce des efforts progressifs au fur et à mesure du vissage contre les parois de l'orifice. Lorsque ces efforts deviennent trop importants par rapport à l'effort de vissage, la vis tronconique se bloque.

Par ailleurs, ladite plaque présente un autre orifice taraudé pratiqué en bordure dudit évidement dans une position diamétralement opposée audit au moins un orifice taraudé de façon à autoriser le blocage de la broche dans deux situations. En effet, comme on l'a expliqué ci-dessus, la couronne peut être maintenue dans une position inclinée par rapport à la plaque ce qui est une caractéristique avantageuse de l'invention. Aussi, compte tenu de cette inclinaison, une portion plus ou moins importante de couronne viendra s'étendre en regard des ouvertures axiales des orifices taraudés. Aussi, on choisira d'introduire la vis à travers l'orifice taraudé, pour lequel une portion plus importante de couronne s'étend au regard de son ouverture axiale. De la sorte, la vis exercera un meilleur appui contre la couronne car elle s'appliquera sur une plus grande surface de couronne. Bien évidemment, la plaque conforme à l'invention n'est nullement limitée à deux orifices taraudés, mais elle peut en comporter plusieurs autres.

Selon un mode de mise en oeuvre de l'invention avantageux, ledit orifice taraudé, préférentiellement de symétrie cylindrique, s'étend sensiblement perpendiculairement à ladite plaque de façon que la vis puisse venir exercer des efforts importants sur la couronne sans risquer son desserrage.

Selon un mode de réalisation particulier, ledit orifice taraudé présente un axe de symétrie sensiblement incliné par rapport à une perpendiculaire à ladite plaque, de manière à ce que ladite vis tronconique présente une portion étendue à l'intérieur dudit évidement, dont la génératrice est perpendiculaire à ladite plaque. De la sorte, les efforts qu'exerce ladite portion étendue contre la couronne sont répartis de manière plus symétriques, et le risque de desserrage est diminué.

En outre, et selon une variante d'exécution particulièrement avantageuse, ledit orifice taraudé présente un axe de symétrie sensiblement tangent audit bord interne de telle manière que l'orifice taraudé apparaisse comme un demi cylindre à base circulaire fendu axialement. De la sorte, lorsqu'une vis tronconique est vissée dans l'orifice taraudé alors qu'elle prend appui contre la couronne située dans l'évidement de la plaque, elle tend à sortir latéralement de son orifice à travers l'ouverture axiale en exerçant des efforts contre la couronne, et elle ne se bloque pas par coincement dans l'orifice. En revanche, les efforts exercés sur la couronne sont plus aisément modulables en fonction du vissage de la vis.

De plus, et dans le but d'augmenter la surface d'appui de la couronne contre le bord interne malgré son inclinaison, ledit bord interne dudit évidement est concave de façon à former un anneau sphérique, tandis que ladite couronne définit un segment sphérique pour venir former rotule à l'intérieur dudit évidement. Cette augmentation des surfaces d'appui permet non seulement un ajustement plus aisé mais aussi, une meilleure rigidité de la liaison entre la couronne et la plaque.

De plus, ladite couronne est avantageusement réalisée dans un métal mou par rapport à ladite plaque, mais aussi par rapport à la vis, de façon que ladite vis vienne tarauder ladite couronne lorsqu'elle est vissée à force dans ledit orifice taraudé. Ainsi, la vis s'appuie non seulement contre la couronne, mais ses filets viennent s'incruster dans le matériau de la couronne ce qui accroît le maintien en position fixe de la couronne par rapport à la plaque.

Préférentiellement, ladite plaque présente au moins un évidement supplémentaire espacé dudit évidement, ledit au moins un évidement supplémentaire étant destiné à recevoir d'autres moyens de fixation pour maintenir une autre broche d'une manière identique à celle décrite ci-dessus.

D'autres particularités et avantages de l'invention ressortiront à la lecture de la description faite ci-après d'un mode de réalisation particulier de l'invention, donné à titre indicatif mais non limitatif, en référence aux dessins annexés sur lesquels :
- la Figure 1 est une vue schématique partielle en élévation de dessus du dispositif d'ostéosynthèse conforme à l'invention ;
- la Figure 2 est une vue schématique en coupe du dispositif d'ostéosynthèse représenté sur la Figure 1, selon le plan II-II ;
- la Figure 3 est une vue schématique en coupe du dispositif d'ostéosynthèse représenté sur la Figure 1, selon le plan III-III.

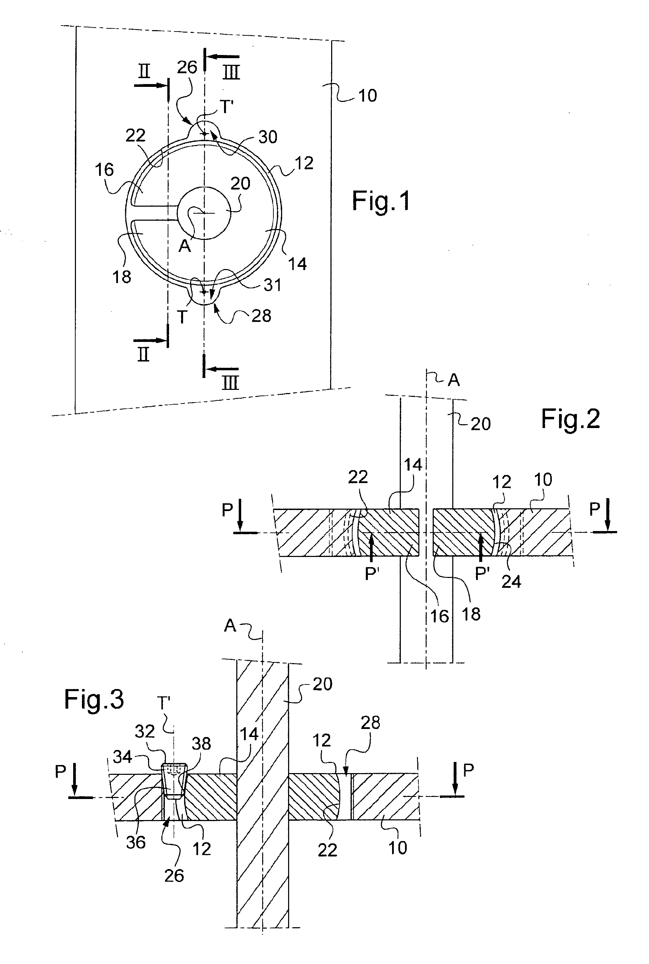

La Figure 1 illustre partiellement une plaque d'ostéosynthèse 10 dans laquelle est ménagé un évidement circulaire 12 qui la traverse de part en part. Une telle plaque comporte bien évidemment une pluralité d'évidements circulaires ménagés de proche en proche dans sa longueur. À l'intérieur de l'évidement 12 est installé une couronne circulaire 14 fendue radialement de manière à former deux extrémités libres en regard 16,18. En outre, à l'intérieur de la couronne 14 est montée coulissante, une broche 20 de section circulaire. Sur la Figure 2, on retrouve la plaque d'ostéosynthèse 10 dans l'épaisseur de laquelle est ménagé l'évidement circulaire 12. La couronne 14 est installée à l'intérieur de cet évidement circulaire 12, tandis que la broche 20, d'axe longitudinal A, est montée à coulissement dans la couronne 14 et orientée selon une direction sensiblement perpendiculaire au plan P défini par la plaque 10. La couronne 14 installée dans la plaque 10 permet également de guider la broche 20 en translation dans une pièce osseuse lorsque le dispositif est installé sur un patient. On observera sur la Figure, que le plan P' défini par la couronne 14 est confondu avec le plan P de la plaque. Or, lorsqu'un tel dispositif est installé sur le patient, ces deux plans sont généralement inclinés l'un par rapport à l'autre, car la couronne 14 est sensiblement pivotée de manière à maintenir la broche 20 inclinée par rapport à la plaque 10.

L'évidement circulaire 12 définit un bord interne 22 fermé dont la surface concave s'étend à la fois selon l'épaisseur de la plaque 10 et sur toute la circonférence de l'évidement 12. Par ailleurs, et en correspondance, la couronne 12 présente un bord externe 24 convexe de telle manière que la couronne puisse former rotule à l'intérieur de l'évidement 12 comme on l'expliquera ci-après lors du montage du dispositif.

On reviendra à présent à la Figure 1, où la plaque 10 présente deux orifices taraudés diamétralement opposés 26, 28. Ces orifices sont pratiqués sensiblement perpendiculairement à la plaque 10 et leur axe de taraudage T, T' est tangent à l'évidement 12 de sorte qu'ils forment deux logements en U dans le bord interne 22. Ces orifices taraudés 26, 28 définissent respectivement une ouverture axiale 30, 31 qui débouche dans l'évidement 12.

Selon un mode particulier de réalisation de l'invention, la broche 20 présente un diamètre compris entre 0,8 et 5 mm, par exemple 4 mm, tandis que la plaque 10 présente une épaisseur comprise entre 3 et 7 mm. L'évidement circulaire 12 présente en conséquence un diamètre compris entre 2,5 mm et 15 mm. Ces éléments sont par exemple réalisés en acier inoxydable du type 316L, l'un des aciers les plus utilisés en chirurgie orthopédique. En revanche, la couronne 14 est elle, réalisée dans un acier inoxydable moins dur.

On se rapportera à présent à la Figure 3 afin de décrire précisément le mode de fonctionnement du dispositif d'ostéosynthèse conforme à l'invention. On retrouve sur cette Figure vue en coupe, la plaque d'ostéosynthèse 10, la broche 20 engagée dans la couronne 14 qui est elle-même engagée dans l'évidement 12. En revanche, à la différence de la Figure 2, un élément supplémentaire, une vis tronconique 32 réalisée dans un acier chirurgical plus dur que celui dans lequel est réalisée la couronne 14, a été au moins partiellement vissée dans l'orifice taraudé 26. La vis tronconique 32 présente, une tête élargie 34 dans laquelle est ménagée axialement une empreinte à six pans pour recevoir un outil de vissage, et une pointe 36 engagée dans l'orifice taraudé 36 et en appui latéralement contre une première portion 38 du bord externe 24 de la couronne 14.

Ainsi, une seconde portion de bord externe 24, diamétralement opposée à la première portion de bord 38, est en appui contre une portion de bord interne 22 diamétralement opposé à la vis tronconique 32. Dans cette position, la couronne 14 est prisonnière à l'intérieur de l'évidement circulaire 12. En revanche la vis tronconique 32 n'étant pas entièrement engagée dans l'orifice taraudé 26, la couronne 14 peut alors pivoter à l'intérieur de l'évidement circulaire et dans des directions perpendiculaires au plan P de la plaque 10, et partant, l'inclinaison de la broche 20 par rapport à la plaque 10 peut être ajustée avec une amplitude relativement importante. C'est là, un avantage de l'invention. Lorsque l'ajustement est réalisé, la vis tronconique 32 est alors entraînée à force en rotation et elle pénètre entièrement dans l'épaisseur de la plaque, à travers l'orifice taraudé 26 et en appui contre la couronne 14. De la sorte, au fur et à mesure que la tête élargie 34 s'enfonce, la couronne est entraînée latéralement en appui à force contre la portion de bord latéral 22, opposée à la vis tronconique 32. Parallèlement, la couronne est resserrée, ses deux extrémités libres en regard 16, 18 se rapprochant l'une de l'autre, de manière à enserrer puis à bloquer la broche 20 en position fixe par rapport à la couronne 14. Aussi, la couronne 14 étant est elle-même en prise entre le bord interne 22 et la vis tronconique 32 vissée dans l'orifice taraudé 26, la broche 20 est par là même maintenue en position fixe par rapport à la plaque 10.

En outre, la couronne 14 étant faite d'un matériau plus mou que le matériau dans lequel est taillée la vis tronconique 32 et découpée la plaque 10, les filets de la vis tronconique viennent découper et s'incruster dans le bord externe 24 de la couronne 14 au fur et à mesure de son vissage. La vis étant alors ancrée dans la couronne 14, cette dernière devient parfaitement solidaire de la plaque 10.

Une plaque du dispositif d'ostéosynthèse conforme à l'invention, est destinée à être installée le long d'une pièce osseuse fracturée et à recevoir une pluralité de broche du type précité pour maintenir en position fixe les différentes parties de pièces osseuses fracturées, par rapport à ladite plaque et par conséquent, pour les maintenir en position fixe les unes par rapport aux autres.

## Revendications

1. Dispositif d'ostéosynthèse comprenant une plaque (10) apte à être étendue le long d'une pièce osseuse et des moyens de fixation pour maintenir une broche (20) en travers de ladite plaque, ladite broche (20) étant vissable dans ladite pièce osseuse, ladite plaque (20) présentant un évidement (12) pratiqué dans son épaisseur pour recevoir lesdits moyens de fixation, ledit évidement définissant un bord interne (22) fermé, lesdits moyens de fixation comprenant une couronne (14) destinée à recevoir ladite broche (20), ladite couronne (14) étant adaptée à venir en prise avec ledit bord interne (22), et lesdits moyens de fixation comprenant en outre, des moyens de blocage vissables (32) pour bloquer ladite broche (20) en position fixe par rapport à ladite plaque (10) ;
**caractérisé en ce que** ladite plaque (10) présente au moins un orifice taraudé (26) pratiqué en bordure dudit évidement (12) de manière à présenter une ouverture axiale (30, 31) débouchant dans ledit bord interne (22), tandis que ladite couronne (14) est fendue radialement de manière à être diamétralement compressible;
et **en ce que** lesdits moyens de blocage vissables comprennent une vis (32) destinée à être vissée à travers ledit orifice taraudé (26) pour venir prendre appui contre ladite couronne (14) à travers ladite ouverture axiale (30, 31) et provoquer à la fois, l'appui à force de ladite couronne (14) contre une portion dudit bord interne (22) diamétralement opposée audit orifice taraudé (26), et le resserrement de ladite couronne (14) sur ladite broche (20), par quoi ladite broche (20) est maintenue en position fixe par rapport à ladite plaque (10).

2. Dispositif d'ostéosynthèse selon la revendication 1, **caractérisé en ce que** ladite vis (32) est tronconique.

3. Dispositif d'ostéosynthèse selon la revendication 1 ou 2, **caractérisé en ce que** ladite plaque (10) présente un autre orifice taraudé (28) pratiqué en bordure dudit évidement (12) dans une position diamétralement opposée audit au moins un orifice taraudé (26).

4. Dispositif d'ostéosynthèse selon l'une quelconque des revendications 1 à 3, **caractérisé en ce que** ledit orifice taraudé (26, 28) s'étend sensiblement perpendiculairement à ladite plaque (10).

5. Dispositif d'ostéosynthèse selon l'une quelconque des revendications 1 à 3,
**caractérisé en ce que** ledit orifice taraudé (26, 28) présente un axe de symétrie sensiblement incliné par rapport à une perpendiculaire à ladite plaque (10), de manière à ce que ladite vis (32) présente une portion étendue à l'intérieur dudit évidement, dont la génératrice est perpendiculaire à ladite plaque (10).

6. Dispositif d'ostéosynthèse selon l'une quelconque des revendications 1 à 5, **caractérisé en ce que** ledit orifice taraudé (26, 28) présente un axe de symétrie (T', T) sensiblement tangent audit bord interne (22).

7. Dispositif d'ostéosynthèse selon l'une quelconque des revendications 1 à 6, **caractérisé en ce que** ledit bord interne (22) dudit évidement (12) est concave de façon à former un anneau sphérique, tandis que ladite couronne (14) définit un segment sphérique pour venir former rotule à l'intérieur dudit évidement (12).

8. Dispositif d'ostéosynthèse selon l'une quelconque des revendications 1 à 7, **caractérisé en ce que** ladite couronne (14) est réalisée dans un métal mou par rapport à ladite plaque (10) de façon que ladite vis tronconique (32) viennent tarauder ladite couronne lorsqu'elle est vissée dans ledit orifice taraudé (26, 28).

9. Dispositif d'ostéosynthèse selon l'une quelconque des revendications 1 à 8, **caractérisé en ce que** ladite plaque (10) présente au moins un évidement supplémentaire espacé dudit évidement, ledit au moins un évidement supplémentaire étant destiné à recevoir d'autres moyens de fixation pour maintenir une autre broche.

## Patentansprüche

1. Osteosynthesevorrichtung, umfassend eine Platte (10), die geeignet ist, entlang eines Knochenteils gelegt zu werden, und Befestigungsmittel zum Halten eines Stiftes (20) durch die Platte, wobei der Stift (20) in das Knochenteil einschraubbar ist, wobei die Platte (20) eine Aussparung (12) aufweist, die in ihrer Dicke hergestellt ist, um die Befestigungsmittel auszunehmen, wobei die Aussparung eine geschlossene Innenkante (22) bestimmt, wobei die Befestigungsmittel eine Krone (14) umfassen, die dazu bestimmt ist, den Stift (20) aufzunehmen, wobei die Krone (14) angepasst ist, mit der Innenkante (22) in Eingriff zu gelangen, und wobei die Befestigungsmittel überdies schraubbare Blockierungsmittel (32) zum Blockierten des Stifts (20) in einer gegenüber der Platte (10) festen Position umfassen;
**dadurch gekennzeichnet, dass** die Platte (10) mindestens eine Gewindeöffnung (26) aufweist, die am Rand der Aussparung (12) hergestellt ist, derart, dass sie eine axiale Öffnung (30, 31) aufweist, die in die Innenkante (22) mündet, während die Krone (14) radial geschlitzt ist, um diametral zusammendrückbar zu sein;
und dadurch, dass die schraubbaren Blockierungsmittel eine Schraube (32) umfassen, die dazu bestimmt ist, durch die Gewindeöffnung (26) geschraubt zu werden, um gegen die Krone (14) durch die axiale Öffnung (30, 31) zur Auflage zu kommen und gleichzeitig unter Kraftanwendung die Auflage der Krone (14) gegen einen Abschnitt der der Gewindeöffnung (26) diametral entgegengesetzten Innenkante (22) und das Verengen der Krone (14) auf dem Stift (20) zu bewirken, wodurch der Stift (20) in einer gegenüber der Platte (10) festen Position gehalten wird.

2. Osteosynthesevorrichtung nach Anspruch 1, **dadurch gekennzeichnet, dass** die Schraube (32) kegelstumpfförmig ist.

3. Osteosynthesevorrichtung nach Anspruch 1 oder 2, **dadurch gekennzeichnet, dass** die Platte (10) eine Gewindeöffnung (28) aufweist, die am Rand der Aussparung (12) in einer der mindestens einen Gewindeöffnung (26) diametral entgegengesetzten Position hergestellt ist.

4. Osteosynthesevorrichtung nach einem der Ansprüche 1 bis 3, **dadurch gekennzeichnet, dass** die Gewindeöffnung (26, 28) sich im Wesentlichen senkrecht zu der Platte (10) erstreckt.

5. Usteosynthesevorrichtung nach einem der Ansprüche 1 bis 3, **dadurch gekennzeichnet, dass** die Gewindeöffnung (26, 28) eine in Bezug zu einer Senkrechten zu der Platte (10) im Wesentlichen geneigte Symmetrieachse aufweist, derart, dass die Schraube (32) einen sich in das Innere der Aussparung erstreckenden Abschnitt aufweist, dessen Mantellinie senkrecht zu der Platte (10) ist.

6. Osteosynthesevorrichtung nach einem der Ansprüche 1 bis 5, **dadurch gekennzeichnet, dass** die Gewindeöffnung (26, 28) eine Symmetrieachse (T', T) aufweist, die im Wesentlichen tangential zur Innenkante (22) ist.

7. Osteosynthesevorrichtung nach einem der Ansprüche 1 bis 6, **dadurch gekennzeichnet, dass** die Innenkante (22) der Aussparung (12) konkav ist, derart, dass sie einen kugelförmigen Ring bildet, während die Krone (14) ein kugelförmiges Segment bestimmt, um ein Kugelgelenk im Inneren der Aussparung (12) zu bilden.

8. Osteosynthesevorrichtung nach einem der Ansprüche 1 bis 7, **dadurch gekennzeichnet, dass** die Krone (14) aus einem im Vergleich zu der Platte (10) weichen Metall hergestellt ist, derart, dass die kegelstumpfförmige Schraube (32) ein Gewinde in der Krone schneidet, wenn sie in die Gewindeöffnung (26, 28) geschraubt wird.

9. Osteosynthesevorrichtung nach einem der Ansprüche 1 bis 8, **dadurch gekennzeichnet, dass** die Platte (10) mindestens eine zusätzliche Aussparung aufweist, die von der Aussparung beabstandet ist, wobei die mindestens eine zusätzliche Aussparung dazu bestimmt ist, andere Befestigungsmittel zum Halten eines anderen Stiftes aufzunehmen.

## Claims

1. Osteosynthesis device comprising a plate (10) that can extend along a piece of bone and fixing means for holding a pin (20) through said plate, said pin (20) being able to be screwed into said piece of bone, said plate (20) having a recess (12) made in the thickness thereof to receive said fixing means, said recess defining a closed inner edge (22), said fixing means comprising a ring (14) which is intended to receive said pin (20), said ring (14) being capable of engaging with said inner edge (22) and said fixing means further comprising screwable locking means (32) to lock said pin (20) in a fixed position with respect to said plate (10); **characterised in that** said plate (10) has at least one threaded opening (26) which is made at the edge of said recess (12) so as to have an axial aperture (30, 31) opening into said inner edge (22), while said ring (14) is radially split so as to be diametrically compressible; and **in that** said screwable locking means comprise a screw (32) which is intended to be screwed through said threaded opening (26) to press against said ring (14) through said axial aperture (30, 32) and cause both said ring (14) to be pressed forcibly against a portion of said inner edge (22) diametrically opposite said threaded opening (26), and said ring (14) to tighten onto said pin (20), by means of which said pin (20) is held in a fixed position with respect to said plate (10).

2. Osteosynthesis device according to claim 1, **characterised in that** said screw (32) has a frustoconical shape.

3. Osteosynthesis device according to either claim 1 or claim 2, **characterised in that** said plate (10) has another threaded opening (28) which is made at the edge of said recess (12) in a position which is diametrically opposite said at least one threaded opening (26).

4. Osteosynthesis device according to any of claims 1 to 3, **characterised in that** said threaded opening (26, 28) extends substantially perpendicularly to said plate (10).

5. Osteosynthesis device according to any of claims 1 to 3, **characterised in that** said threaded opening (26, 28) has an axis of symmetry that is substantially inclined with respect to a perpendicular to said plate (10), such that said screw (32) has an extended portion inside said recess, the generatrix of which is perpendicular to said plate (10).

6. Osteosynthesis device according to any of claims 1 to 5, **characterised in that** said threaded opening (26, 28) has an axis of symmetry (T', T) which is substantially tangential to said inner edge (22).

7. Osteosynthesis device according to any of claims 1 to 6, **characterised in that** said inner edge (22) of said recess (12) is concave so as to form a spherical annulus, whereas said ring (14) defines a spherical segment to form a ball inside said recess (12).

8. Osteosynthesis device according to any of claims 1 to 7, **characterised in that** said ring (14) is made of a metal that is soft by comparison with said plate (10) such that said frustoconical screw (32) will self-tap into said ring when screwed into said threaded opening (26, 28).

9. Osteosynthesis device according to any of claims 1 to 8, **characterised in that** said plate (10) has at least one additional recess which is at a distance from said recess, said at least one additional recess being intended to receive other fixing means to hold another pin.
